# EUROPÄISCHE PATENTSCHRIFT

(11) **EP 4 151 185 B1**
(45) Veröffentlichungstag und Bekanntmachung des Hinweises auf die Patenterteilung: **07.02.2024**
(21) Anmeldenummer: 22196208.7
(22) Anmeldetag: 16.09.2022
(51) Int. Cl.: A61F 9/007, A61B 17/00

(54) **KONSOLE UND FUSSBEDIENEINHEIT FÜR EIN MEDIZINISCHES BEHANDLUNGSSYSTEM SOWIE MEDIZINISCHES BEHANDLUNGSSYSTEM HIERFÜR**
CONSOLE AND FOOT CONTROL UNIT FOR A MEDICAL TREATMENT SYSTEM AND MEDICAL TREATMENT SYSTEM FOR SAME
CONSOLE ET UNITÉ DE COMMANDE AU PIED POUR UN SYSTÈME DE TRAITEMENT MÉDICAL AINSI QUE SYSTÈME DE TRAITEMENT MÉDICAL CORRESPONDANT

(30) Priorität: 21.09.2021 DE 102021124421
(43) Veröffentlichungstag der Anmeldung: 22.03.2023
(73) Patentinhaber: Carl Zeiss Meditec AG, 07745 Jena (DE)
(72) Erfinder: Wamser, Michael, 73434 Aalen (DE)
(74) Vertreter: Hofstetter, Schurack & Partner

(56) Entgegenhaltungen:
- WO-A1-2014/172550
- US-A1- 2011 087 069
- US-A1- 2018 042 802
- US-A1- 2019 350 757

## Beschreibung

Die Erfindung betrifft eine Konsole für ein medizinisches Behandlungssystem, insbesondere ein ophthalmochirurgisches System zur Behandlung eines Auges, mit einem Konsolengehäuse und einer Steuereinheit zum Steuern eines medizinischen Behandlungsinstruments, wobei eine Fußbedieneinheit drahtlos mit der Steuereinheit koppelbar ist, und das Konsolengehäuse wenigstens eine Seitenwand aufweist. Ferner betrifft die Erfindung eine Fußbedieneinheit für ein medizinisches Behandlungssystem, insbesondere ein ophthalmochirurgisches System zur Behandlung eines Auges, mit einem Gehäuse, einem im Gehäuse angeordneten elektrischen Energiespeicher, einem mittels eines Fußes betätigbaren Stellglied, einer Kommunikationseinheit zum drahtlosen Kommunizieren mit einer Konsole des medizinischen Behandlungssystems sowie einer Aufladeeinheit zum Aufladen des elektrischen Energiespeichers. Schließlich betrifft die Erfindung auch ein medizinisches Behandlungssystem, insbesondere ein ophthalmochirurgisches System, zur Behandlung eines Auges, mit zumindest einem medizinischen Behandlungsinstrument, einer Konsole zum Anschließen und Betreiben des medizinischen Behandlungsinstruments in einem bestimmungsgemäßen Betrieb, einer Fußbedieneinheit zum zumindest teilweisen Steuern des bestimmungsgemäßen Betriebs des medizinischen Behandlungsinstruments, wobei die Fußbedieneinheit drahtlos mit der Konsole koppelbar ist und einen elektrischen Energiespeicher zum Versorgen der Fußbedieneinheit mit elektrischer Energie zumindest während des bestimmungsgemäßen Betriebs des medizinischen Behandlungssystems aufweist.

Medizinische Behandlungssysteme, ophthalmochirurgische Systeme zur Behandlung eines Auges, Konsolen sowie auch Fußbedieneinheiten hierfür sind im Stand der Technik umfänglich bekannt, sodass es diesbezüglich eines gesonderten druckschriftlichen Nachweises nicht bedarf. Medizinische Behandlungssysteme dienen dazu, unterschiedlichste Behandlungen, beispielsweise Operationen, an einem Körper eines Lebewesens, beispielsweise dem Menschen oder einem Tier, durchführen zu können. Hierzu wird häufig ein medizinisches Behandlungsinstrument benutzt, welches manuell oder mittels eines Roboters während einer Operation geführt werden kann. Für den bestimmungsgemäßen Betrieb des medizinischen Behandlungsinstruments ist es häufig erforderlich, das medizinische Behandlungsinstrument mit elektrischer Energie, einem oder mehreren Behandlungsfluiden sowie Betriebsdaten zu versorgen. Diesem Zweck dient in der Regel die Konsole, mit der das medizinische Behandlungsinstrument zumindest während des bestimmungsgemäßen Betriebs gekoppelt werden kann. Die Konsole ist somit zum Anschließen und Betreiben des medizinischen Behandlungsinstruments ausgebildet.

Zu diesem Zweck weist die Konsole in der Regel eine Steuereinheit zum Steuern des medizinischen Behandlungsinstruments auf. Die Steuereinheit steht mit dem medizinischen Behandlungselement in Kommunikationsverbindung, um beispielsweise Steuerdaten an das Behandlungsinstrument zu übermitteln, oder auch Betriebsdaten oder Messdaten vom medizinischen Behandlungsinstrument zu erhalten und auszuwerten. Die Konsole ermöglicht es darüber hinaus, auf die Steuereinheit einzuwirken, um den gewünschten bestimmungsgemäßen Betrieb des medizinischen Behandlungsinstruments realisieren zu können.

Zu diesem Zweck kann die Konsole eine Eingabeeinheit aufweisen, die es beispielsweise einem Operateur ermöglicht, Einstellungen bezüglich des gewünschten bestimmungsgemäßen Betriebs vornehmen zu können. Die Eingabeeinheit kann zum Beispiel dazu ausgebildet sein, dass der Operateur eine manuelle Eingabe vornehmen kann. Manuelle Eingaben sind jedoch bei bestimmten medizinischen Behandlungen nur schlecht oder sogar teilweise sogar unmöglich. Aus diesem Grund kann eine Eingabeeinheit nach Art einer Fußbedieneinheit vorgesehen sein, die abhängig von einer Betätigung mittels eines Fußes ein Steuersignal für die Steuereinheit der Konsole bereitstellt. Die Fußbedieneinheit kann dabei Bestandteil der Konsole sein oder auch als eine separate Einheit ausgebildet sein. Die Fußbedieneinheit kann für den bestimmungsgemäßen Betrieb mit der Konsole in vorgebbarer Weise gekoppelt werden. Häufig sind hierfür eine oder mehrere Leitungen vorgesehen, die beispielsweise der Versorgung der Fußbedieneinheit mit elektrischer Energie und/oder dem Übermitteln von Daten beziehungsweise Signalen dienen. Derartige Leitungen erweisen sich während der Nutzung des medizinischen Behandlungssystems häufig als nachteilig. Aus diesem Grund offenbart zum Beispiel die US 2006/0219049 A1 einen Fußschalter zum Steuern eines Operationssystems. Der Fußschalter detektiert eine mittels eines Fußes vorgenommene mechanische Eingabe und liefert über eine drahtlose Kommunikationseinrichtung ein Steuersignal für das medizinische Instrument.

Darüber hinaus ist es bekannt, dass die Fußbedieneinheit einen eigenen elektrischen Energiespeicher aufweist, der für den bestimmungsgemäßen Betrieb elektrische Energie bereitstellt. Der elektrische Energiespeicher ist in der Regel durch einen aufladbaren Akkumulator gebildet. In diesem Zusammenhang offenbart zum Beispiel die US 2004/0212344 A1 ein Gerät und ein Verfahren zur Wartung einer Defibrillatorbatterieladung und optionalen Kommunikation. Ferner offenbart die US 8,565,839 B2 ein Leistungsmanagement für drahtlose Geräte.

Ophthalmochirurgische Systeme, sowie auch Verfahren zu deren Betrieb, sind im Stand der Technik ebenfalls bekannt, sodass es auch diesbezüglich eines gesonderten druckschriftlichen Nachweises nicht bedarf. Zur Behandlung einer Augenlinsentrübung, in der Medizin auch als Grauer Star bezeichnet, sind unterschiedliche chirurgische Techniken bekannt. Am weitesten verbreitet ist die Phakoemulsifikation, bei der eine dünne Hohlnadel in einen Kapselsack, in dem die Augenlinse angeordnet ist, eingeführt und zu Untraschallschwingungen angeregt wird. Mittels der vibrierenden Hohlnadel kann die Linse emulsifiziert werden, wobei hierbei freigesetzte Linsenpartikel über eine Aspirationsleitung mittels einer Pumpe abgesaugt werden können. Dabei wird ein Spülfluid, auch Irrigationsfluid genannt, zugeführt. Die Linsenpartikel werden zusammen mit dem Fluid als Aspirationsfluid abgesaugt. Sobald die Linse vollständig emulsifiziert und entfernt ist, kann in den dann geleerten Kapselsack eine neue künstliche Linse eingesetzt werden. Hierdurch kann für den behandelten Patienten wieder ein gutes Sehvermögen erreicht werden. Während der Durchführung einer derartigen Operation mittels des ophthalmochirurgischen Systems wird als medizinisches Behandlungsinstrument ein Handstück eingesetzt, welches die Hohlnadel aufweist und zumindest während des bestimmungsgemäßen Betriebs an eine entsprechende Konsole des ophthalmochirurgischen Systems angeschlossen ist. Die Konsole stellt dabei das Irrigationsfluid und ebenfalls eine Ableitung des Aspirationsfluids bereit. Darüber hinaus stellt die Konsole auch elektrische Energie für den bestimmungsgemäßen Betrieb des Handstücks bereit. Mittels einer Steuereinheit der Konsole kann der Betrieb des Handstücks gesteuert werden. Da für die durchzuführende Operation die Hand des Operateurs für steuernde Eingaben in der Regel nicht zur Verfügung steht, wird häufig eine Fußbedieneinheit genutzt, um den Betrieb des Handstücks zu steuern. Die Fußbedieneinheit ist hierzu mit der Konsole entsprechend gekoppelt.

Auch wenn der Stand der Technik eine drahtlos mit der Konsole koppelbare Fußbedieneinheit zeigt, verbleiben dennoch Nachteile. Dies betrifft sowohl das Lagern der Fußbedieneinheit in einer Nichtgebrauchsstellung als auch die Zufuhr von elektrischer Energie zum elektrischen Energiespeicher, damit die Fußbedieneinheit für eine folgende Nutzung wieder zur Verfügung steht. In diesem Zusammenhang offenbart zum Beispiel die US 8,565,839 B2 eine induktive Kopplung der Fußbedieneinheit mit einer entsprechenden Ladeeinrichtung. Die Nutzung einer induktiven Ladeeinrichtung erweist sich insofern als nachteilig, als dass konstruktiver Aufwand für die Unterbringung von entsprechenden Spulen in der Fußbedieneinheit und auch in der Ladeeinrichtung erforderlich sind. Die Ladeeinrichtung erfordert darüber hinaus einen aufwendigen Energiewandler. Schließlich ist zu bedenken, dass die induktiv zugeführte Energie auch einen begrenzten Wirkungsgrad nach sich zieht und darüber hinaus Störungen, insbesondere in Bezug auf die elektromagnetische Verträglichkeit, verursachen kann. Die Nutzung einer derartigen Ladeeinrichtung ist daher im bestimmungsgemäßen Betrieb in der Regel nicht unproblematisch.

Aber auch ein leitungsgebundenes Laden der Fußbedieneinheit erweist sich als nachteilig, als dass eine entsprechende leitungsgebundene Energieversorgung sichergestellt werden muss. Dies fordert jedoch wieder den zuvor als ungünstig bereits erkannten Einsatz von elektrischen Leitungen in Form von Kabeln. Schließlich können auch derartige Leitungen hinsichtlich der elektromagnetischen Verträglichkeit ungünstig sein.

Ferner wird auf die Druckschriften US 2011 / 0 087 069 A1, WO 2013 / 134 133 A, WO 2014 / 172 550 A1 und US 2019 / 350 757 A1 verwiesen.

Der Erfindung liegt die Aufgabe zugrunde, die Anordnung der Fußbedieneinheit insbesondere außerhalb des bestimmungsgemäßen Betriebs, insbesondere auch einer Energieversorgung, zu verbessern.

Als Lösung werden mit der Erfindung eine Konsole, eine Fußbedieneinheit sowie ein medizinisches Behandlungssystem gemäß den unabhängigen Ansprüchen vorgeschlagen.

Vorteilhafte Weiterbildungen ergeben sich durch Merkmale der abhängigen Ansprüche.

In Bezug auf eine gattungsgemäße Konsole wird mit der Erfindung insbesondere vorgeschlagen, dass die Seitenwand eine Anlagefläche zum Anordnen der Fußbedieneinheit in einer Nichtgebrauchsstellung aufweist, wobei die Anlagefläche zumindest im Bereich der Anordnung der Fußbedieneinheit gegenüber einer horizontalen Ebene geneigt ist, um beim Anordnen der Fußbedieneinheit an der Anlagefläche durch die Gewichtskraft der Fußbedieneinheit ein Anliegen der Fußbedieneinheit an der Anlagefläche zu bewirken, wobei die Seitenwand im Bereich der Anlagefläche eine konsolenseitige Verbindungseinheit zum lösbaren Verbinden der Fußbedieneinheit mit der Konsole aufweist.

In Bezug auf eine gattungsgemäße Fußbedieneinheit wird mit der Erfindung insbesondere vorgeschlagen, dass das Gehäuse einen Verbindungsbereich zum lösbaren Anordnen der Fußbedieneinheit in einer Nichtgebrauchsstellung an einer Anlagefläche der Konsole aufweist, wobei der Verbindungsbereich eine fußbedieneinheitsseitige Verbindungseinheit zum lösbaren Verbinden der Fußbedieneinheit mit der Konsole aufweist.

In Bezug auf ein gattungsgemäßes medizinisches Behandlungssystem wird mit der Erfindung insbesondere vorgeschlagen, dass wenigstens die Konsole oder die Fußbedieneinheit gemäß der Erfindung ausgebildet ist.

Die Erfindung basiert unter anderem auf dem Gedanken, dass die Fußbedieneinheit vorzugsweise vollständig kabellos ausgebildet ist und weder im bestimmungsgemäßen Betrieb noch in der Nichtgebrauchsstellung einen Anschluss über ein Kabel benötigt. Zugleich kann insbesondere für die Nichtgebrauchsstellung gewährleistet werden, dass die Fußbedieneinheit an einer vorgegebenen Stelle auf einfache Weise angeordnet ist und somit für die bestimmungsgemäße Nutzung im medizinischen Behandlungssystem auf einfache Weise verfügbar ist. Dabei ermöglicht es die geneigte Anlagefläche der Konsole, dass die Fußbedieneinheit im an der Konsole angeordneten Zustand bereits aufgrund ihrer Gewichtskraft an der Anlagefläche der Konsole anliegt und somit eine zuverlässige Verbindung zwischen der Konsole und der Fußbedieneinheit erreicht werden kann, sodass außerhalb des bestimmungsgemäßen Betriebs die Konsole zusammen mit der Fußbedieneinheit verfahren werden kann. Die Fußbedieneinheit kann durch ihre Verbindung mit der Konsole mitgeführt werden. Es kann daher eine Handhabungseinheit geschaffen werden, die die Nutzung vereinfacht. Es brauchen für die Fußbedieneinheiten keine separaten Ablageplätze mehr bereitgestellt zu werden. Die Bereitstellung einer Konsole für eine medizinische Behandlung erlaubt es zugleich auch, die Fußbedieneinheit bereitzustellen. Besonders vorteilhaft ermöglicht es die Erfindung, eine kabellose Fußbedieneinheit einer Konsole individuell zuzuordnen. Dadurch können auch funktionsspezifische Besonderheiten eines jeweiligen medizinischen Behandlungssystems auf einfache Weise zuverlässig realisiert werden. Insgesamt ermöglicht es die Erfindung somit, die Handhabung einer kabellosen Fußbedieneinheit zu verbessern.

Die Seitenwand des Gehäuses, die die Anlagefläche bereitstellt, kann als geschlossene Seitenwand ausgebildet sein. Darüber hinaus kann sie jedoch auch zumindest teilweise eine oder mehrere Durchgangsöffnungen aufweisen. Die Durchgangsöffnungen können auch im Bereich der Anlagefläche vorgesehen sein. Das Gehäuse, insbesondere die Seitenwand, kann aus einem geeigneten stabilen Werkstoff gebildet sein, beispielsweise Metall, Kunststoff, einen Verbundwerkstoff, Kombinationen hiervon und/oder dergleichen. Dabei hat ein elektrisch leitfähiges Gehäuse den Vorteil, dass einerseits konsolenseitig eine Aussendung von Störungen in Bezug auf elektromagnetische Verträglichkeit geringgehalten oder sogar vermieden werden kann und andererseits eine Beeinflussung der Konsole, insbesondere der Steuereinheit, durch elektromagnetische Signale von außerhalb der Konsole gedämpft beziehungsweise unterdrückt werden kann. Die Zuverlässigkeit der Funktion des medizinischen Behandlungssystems kann dadurch verbessert werden.

Die Seitenwand weist im Bereich der Anlagefläche die konsolenseitige Verbindungseinheit zum lösbaren Verbinden der Fußbedieneinheit mit der Konsole auf. Zu diesem Zweck kann beispielsweise in einem unteren Bereich der Anlagefläche eine Halteleiste angeordnet sein. Die Halteleiste kann auch mit weiteren Verbindungselementen wie Stiften, Ausnehmungen und dergleichen kombiniert sein.

Aufgrund der geneigten Anlagefläche braucht die Fußbedieneinheit nicht nach Art einer Aufnahmetasche aufgenommen zu sein. Dadurch ist die Fußbedieneinheit besonders einfach verfügbar und mit der Konsole verbindbar. Die Neigung der Anlagefläche gegenüber der horizontalen Ebene ist vorzugsweise derart, dass die Anlagefläche der horizontalen Ebene im Bereich des Fußes der Konsole abgewandt ist. Dabei reicht bereits eine geringfügige Neigung gegenüber der Vertikalen aus, die beispielsweise in einem Bereich von etwa 3° bis etwa 15°, bevorzugt in einem Bereich von etwa 5° bis etwa 10°, liegen kann.

Die Konsole weist eine Ladeeinheit zum Aufladen eines elektrischen Energiespeichers der Fußbedieneinheit auf, wobei die Anlagefläche einen Ladeanschluss mit wenigstens zwei elektrischen Kontakten zum Kontaktieren von entsprechenden Gegenkontakten der Fußbedieneinheit aufweist. Hierdurch kann erreicht werden, dass der Energiespeicher der Fußbedieneinheit in der Nichtgebrauchsstellung, in der die Fußbedieneinheit an der Konsole angeordnet ist, aufgeladen werden kann. Dadurch kann die Fußbedieneinheit also ergänzend für den bestimmungsgemäßen Gebrauch betriebsbereit gemacht beziehungsweise betriebsbereit gehalten werden. Dies kann erreicht werden, ohne dass zusätzliche elektrische Verbindungsmittel zu betätigen wären, um ein Aufladen des elektrischen Energiespeichers über die Konsole zu ermöglichen. Lediglich die Anordnung der Fußbedieneinheit an der Anlagefläche der Konsole kann zugleich auch eine elektrische Kopplung zwischen der Ladeeinheit der Konsole und dem elektrischen Energiespeicher der Fußbedieneinheit realisieren. Die Ladeeinheit kann dem Grunde nach lediglich durch eine elektrische Verbindung mit einer elektrischen Energiequelle wie dem öffentlichen Energieversorgungsnetz oder dergleichen gebildet sein. Vorzugsweise umfasst die Ladeeinheit jedoch auch einen Energiewandler, um dem Energiespeicher bedarfsgerecht elektrische Energie zuführen zu können. Eine Energiewandlung in der Fußbedieneinheit kann dadurch einspart werden. Der Energiewandler kann beispielsweise eine Spannungsanpassung zwischen der Spannungsquelle und dem Energiespeicher realisieren. Zu diesem Zweck sind wenigstens zwei elektrische Kontakte im Bereich der Anlagefläche vorgesehen, sodass bei einer Anordnung der Fußbedieneinheit an der Konsole zugleich auch ein elektrischer Anschluss der Fußbedieneinheit an die Konsole, insbesondere an deren Ladeeinheit, realisiert werden kann. Zu diesem Zweck weist die Fußbedieneinheit entsprechende Gegenkontakte auf. Die elektrischen Kontakte können beispielsweise als Stiftkontakte ausgebildet sein, die aus der Ebene der Anlagefläche nach außen hervorstehen. Die elektrischen Kontakte können beispielsweise federnd gelagert sein. Natürlich können auch weitere Kontakte vorgesehen sein, beispielsweise um die Fußbedieneinheit, insbesondere deren korrekte Position an der Anlagefläche, detektieren zu können.

Die Anlagefläche weist einen aus der Anlagefläche hervorragenden umlaufenden Kragen auf, der die elektrischen Kontakte umgibt. Der Kragen kann aus dem gleichen Werkstoff wie die Seitenwand gebildet sein. Der Kragen kann einstückig mit der Seitenwand ausgebildet sein. Der Kragen kann alternativ aber auch als separates Bauteil ausgebildet sein, welches an der Seitenwand an der vorgegebenen Position befestigt ist. In Umfangsrichtung kann der Kragen eine vorgebbare innere und/oder äußere Kontur aufweisen. Der Kragen weist vorzugsweise eine Höhe auf, die mindestens der maximalen Höhe der elektrischen Kontakte entspricht. Dadurch können die elektrischen Kontakte vor äußeren mechanischen Einwirkungen und besonders vor Fluid wie zum Beispiel Aspirationsfluid oder Irrigationsfluid geschützt werden, welches an der Seitenwand nach unten ablaufen kann. Der Kragen bewirkt somit einen guten Schutz der elektrischen Kontakte vor Korrosion, wodurch für lange Zeit eine zuverlässige Funktion der elektrischen Kontakte sichergestellt werden kann.

Die Verbindungseinheit weist wenigstens zwei Haltestifte zum Anordnen in entsprechenden Aufnahmeöffnungen der Fußbedieneinheit auf, die voneinander zumindest horizontal beabstandet angeordnet sind. Durch die Haltestifte kann eine verbesserte Halterung der Fußbedieneinheit an der Konsole erreicht werden. Insbesondere kann ein besonders einfaches Anordnen an der Konsole sowie Abnehmen von der Konsole erreicht werden. Die Haltestifte sind vorzugsweise derart hinsichtlich ihrer Länge bemessen, dass sie die Fußbedieneinheit im an der Konsole angeordneten Zustand nicht durchragen. Die Haltestifte können eine vorgebbare Kontur in Bezug auf einen Querschnitt aufweisen, die an eine Innenkontur der Aufnahmeöffnungen der Fußbedieneinheit angepasst ausgebildet ist. Dadurch kann eine Codierung erreicht werden, sodass nicht jede beliebige Fußbedieneinheit mit jeder beliebigen Konsole kombiniert werden kann. Dies kann natürlich auch durch das Anordnen der Haltestifte, die Anzahl der Haltestifte oder dergleichen erreicht werden. Vorzugsweise sind die Haltestifte im Wesentlichen gleich ausgebildet. Darüber hinaus ist es bevorzugt, wenn die Haltestifte im Wesentlichen senkrecht aus der Auflagefläche herausragen. Die Haltestifte können aus dem gleichen Werkstoff wie die Seitenwand gebildet sein. Sie können insbesondere einstückig mit der Seitenwand ausgebildet sein. Es kann aber auch vorgesehen sein, dass die Haltestifte separate Teile sind, die mit der Seitenwand verbunden werden können, beispielsweise mittels einer Schraubverbindung, einer Nietverbindung und/oder dergleichen.

Es wird ferner vorgeschlagen, dass die elektrischen Kontakte als federnde elektrische Stiftkontakte ausgebildet sind. Dadurch kann auch bei variablen Positionen der Gegenkontakte der Fußbedieneinheit ein zuverlässiger elektrischer Kontakt gewährleistet werden.

Bezüglich der Fußbedieneinheit ist vorgesehen, dass der Verbindungsbereich wenigstens zwei Aufnahmeöffnungen zum Aufnehmen von entsprechenden Haltestiften der Konsole aufweist, die voneinander zumindest horizontal beabstandet angeordnet sind. Hierdurch kann eine einfach zu bedienende Anordnung der Fußbedieneinheit an der Konsole beziehungsweise Abnahme von der Konsole erreicht werden. Durch die in die Aufnahmeöffnungen aufgreifenden Haltestifte kann ferner erreicht werden, dass die Fußbedieneinheit im an der Konsole angeordneten Zustand gegen Verdrehen aufgrund der Einwirkung äußerer Kräfte geschützt werden kann.

Die Aufladeeinheit weist einen Aufladeanschluss mit wenigstens zwei am Verbindungsbereich angeordneten elektrischen Gegenkontakten zum Kontaktieren von entsprechenden elektrischen Kontakten der Konsole auf. Dadurch kann im an der Konsole angeordneten Zustand eine elektrische Verbindung hergestellt werden, die es erlaubt, den elektrischen Energiespeicher aufzuladen. Die Aufladeeinheit kann dem Grunde nach lediglich durch eine elektrische Verbindung gebildet sein. Darüber hinaus kann die Aufladeeinheit jedoch auch einen elektrischen Energiewandler aufweisen, mittels dem eine Anpassung einer elektrischen Spannung an den elektrischen Energiespeicher erreicht werden kann. Die Aufladeeinheit kann für eine unidirektionale Energiezuführung ausgebildet sein. Die Aufladeeinheit ist vorzugsweise elektrisch mit dem elektrischen Energiespeicher gekoppelt.

Ferner wird vorgeschlagen, dass der Verbindungsbereich ein Plateau zum Anordnen in einer durch den Kragen bereitgestellten Ausnehmung aufweist, wobei die elektrischen Gegenkontakte im Bereich einer Plateauebene des Plateaus angeordnet sind. Dadurch kann erreicht werden, dass die elektrische Verbindung im an der Konsole angeordneten Zustand vor äußeren Einwirkungen geschützt ist. Vorteilhaft ist dies insbesondere auch dann, wenn während eines Aufladevorgangs des elektrischen Energiespeichers eine Betriebsspannung zwischen den elektrischen Kontakten größer als eine Kleinspannung im Sinne der Normung ist. Die Kleinspannung kann zum Beispiel der Richtlinie 2001/95/EG des Europäischen Parlaments und des Rates vom 3. Dezember 2001 über die allgemeine Produktsicherheit entsprechen. Vorzugsweise handelt es sich hierbei um eine Wechselspannung, deren Effektivwert kleiner als etwa 50 V ist, oder um eine Gleichspannung, die kleiner als etwa 75 V ist.

Durch die Anordnung der elektrischen Gegenkontakte auf der Plateauoberfläche sind diese besonders gut für etwaige Reinigungsprozesse zugänglich. Auch für die elektrischen Gegenkontakte kann vorgesehen sein, dass diese federnd gelagert sind. Die elektrischen Gegenkontakte sind vorzugsweise durch flächige, elektrisch leitfähige Elemente gebildet, die einen guten elektrischen Kontakt zu den elektrischen Kontakten der Konsole herstellen können. Besonders vorteilhaft sind sie zum Kontaktieren von Kontaktstiften ausgebildet.

Die für die erfindungsgemäße Konsole und die erfindungsgemäße Fußbedieneinheit angegebenen Vorteile und Wirkungen gelten natürlich gleichermaßen auch für das mit der erfindungsgemäßen Konsole und der erfindungsgemäßen Fußbedieneinheit ausgerüstete medizinische Behandlungssystem und umgekehrt.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Fig. nicht explizit gezeigt und erläutert sind, jedoch durch separierte Merkmalskombinationen aus den erläuterten Ausführungen hervorgehen und erzeugbar sind.

Es zeigen:
- Fig. 1: eine schematische Darstellung eines Ausführungsbeispiels eines ophthalmochirurgischen Systems mit einer Konsole, einer Fußbedieneinheit sowie einem an die Konsole angeschlossenen Handstück,
- Fig. 2: eine schematisch-perspektivische Darstellung der Konsole gemäß Fig. 1,
- Fig. 3: eine schematisch-perspektivische Darstellung einer Seitenwand in einem unteren Bereich der Konsole gemäß Fig. 1,
- Fig. 4: eine vergrößerte Darstellung eines Bereichs IV aus Fig. 3,
- Fig. 5: eine schematische Draufsicht auf eine Rückseite der Fußbedieneinheit gemäß Fig. 1 im Bereich von elektrischen Gegenkontakten,
- Fig. 6: eine schematische Seitenansicht eines Ausschnitts einer von der Konsole im Bereich der Anlagefläche beabstandet positionierten Fußbedieneinheit,
- Fig. 7: eine schematisch-perspektivische Darstellung wie Fig. 3, wobei die Fußbedieneinheit an der Konsole angeordnet ist.

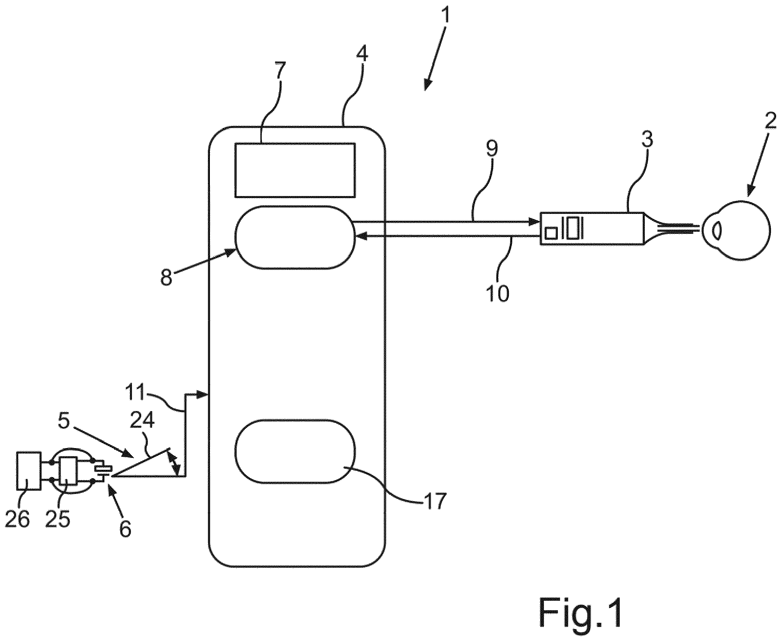

Fig. 1 zeigt in einer schematischen Blockschaltbilddarstellung ein ophthalmochirurgisches System 1 als medizinisches Behandlungssystem, welches zur Behandlung eines Auges 2 dient. Das ophthalmochirurgische System 1 weist ein medizinisches Behandlungsinstrument auf, welches vorliegend durch ein Handstück 3 gebildet ist. Ferner weist das ophthalmochirurgische System 1 eine Konsole 4 zum Anschließen und Betreiben des Handstücks 3 in einem bestimmungsgemäßen Betrieb auf. Weiterhin weist das ophthalmochirurgische System 1 eine Fußbedieneinheit 5 auf, die vorliegend als Fußpedal ausgebildet ist. Die Fußbedieneinheit 5 ist drahtlos über eine Funkverbindung 11 mit der Konsole 4 koppelbar und weist einen elektrischen Energiespeicher 6 zum Versorgen der Fußbedieneinheit 5 mit elektrischer Energie zumindest während des bestimmungsgemäßen Betriebs des Handstücks 3 auf.

Ferner weist die Konsole 4 eine Steuereinheit 7 sowie fluidische und elektrische Versorgungseinheiten 8 auf, die unter anderem dazu dienen, dem Handstück 3 über eine Irrigationsleitung 9 ein Irrigationsfluid zuzuführen und über eine Aspirationsleitung 10 ein Aspirationsfluid vom Handstück 3 abzuführen.

Die Fußbedieneinheit 5 ist vorliegend kabellos ausgebildet, weshalb sie zumindest während des bestimmungsgemäßen Betriebs vom elektrischen Energiespeicher 6 mit elektrischer Energie versorgt wird. Darüber hinaus ist die Fußbedieneinheit 5 im bestimmungsgemäßen Betrieb über die drahtlose Funkverbindung 11 mit der Steuereinheit 7 in Kommunikationsverbindung, sodass ein Betriebszustand des Handstücks 3 zumindest teilweise eingestellt werden kann.

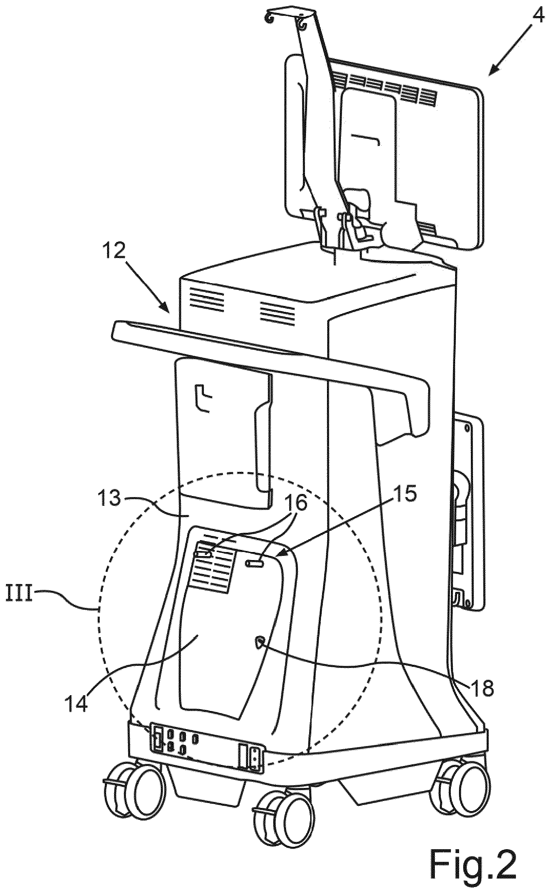

Fig. 2 zeigt die Konsole 4 gemäß Fig. 1 in einer schematisch-perspektivischen Ansicht, die die Rückseite umfasst. Die Konsole 4 weist ein Konsolengehäuse 12 auf, in dem die Elemente beziehungsweise Einheiten der Konsole 4 angeordnet sind. Das Konsolengehäuse 12 weist eine Seitenwand 13 auf, die vorliegend eine Rückseite der Konsole 4 bildet. Die Seitenwand 13 weist eine Anlagefläche 14 auf, die zum Anordnen der Fußbedieneinheit 5 in einer Nichtgebrauchsstellung dient. Die Anlagefläche 14 ist im Bereich der Anordnung der Fußbedieneinheit 5 gegenüber einer horizontalen Ebene geneigt, um beim Anordnen der Fußbedieneinheit 5 an der Anlagefläche 14 durch die Gewichtskraft der Fußbedieneinheit 5 ein Anliegen der Fußbedieneinheit 5 an der Anlagefläche 14 zu bewirken. Die Anlagefläche 14 ist vorliegend eben beziehungsweise flach, insbesondere ungekrümmt ausgebildet.

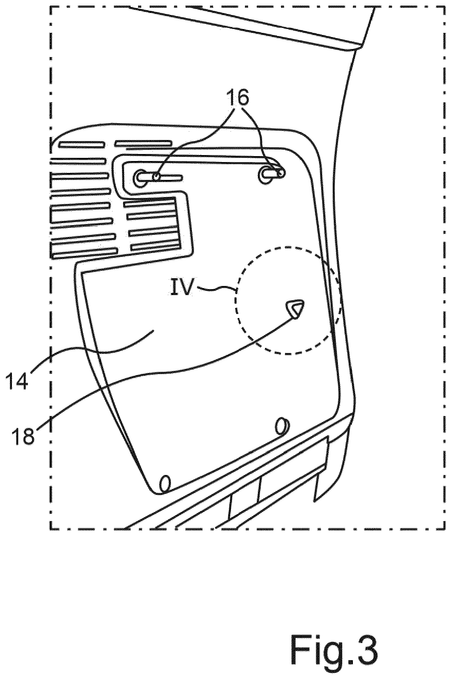

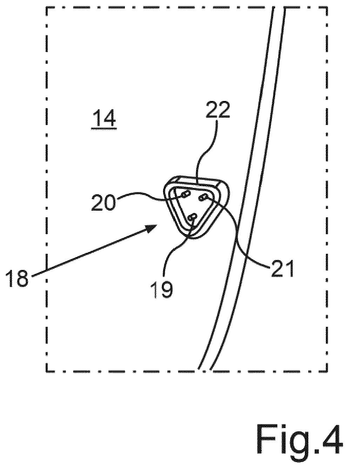

Um die Fußbedieneinheit 5 im an der Konsole 4 angeordneten Zustand halten zu können, weist die Seitenwand 13 im Bereich der Anlagefläche 14 eine konsolenseitige Verbindungseinheit 15 zum lösbaren Verbinden der Fußbedieneinheit 5 mit der Konsole 4 auf. Zu diesem Zweck weist die konsolenseitige Verbindungseinheit 15 zwei Haltestifte 16 zum Anordnen in entsprechenden Aufnahmeöffnungen der Fußbedieneinheit 5 auf, die voneinander horizontal beabstandet angeordnet sind. Vorliegend ist vorgesehen, dass die Haltestifte 16 im Wesentlichen etwa auf der gleichen Höhe gegenüber einem horizontalen Boden angeordnet sind. In alternativen Ausgestaltungen kann dies jedoch auch abweichen. Fig. 3 zeigt eine entsprechende vergrößerte Darstellung eines Ausschnitts III in Fig. 2. Fig. 4 zeigt in einer schematischen Draufsicht eine vergrößerte Darstellung eines Bereichs IV in Fig. 3. Zu erkennen ist, dass die Anlagefläche 14 einen aus der Anlagefläche 14 herausragenden umlaufenden Kragen 22 aufweist, der elektrische Kontakte 19, 20, 21 umgibt.

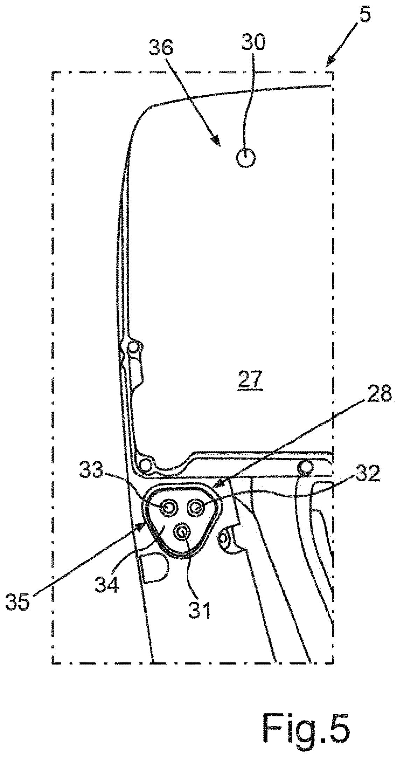

Die Konsole 4 weist ferner eine Ladeeinheit 17 zum Aufladen des elektrischen Energiespeichers 6 der Fußbedieneinheit 5 auf. Die Anlagefläche 14 weist einen Ladeanschluss 18 der Ladeeinheit 17 mit drei elektrischen Kontakten 19, 20, 21 zum Kontaktieren von entsprechenden elektrischen Gegenkontakten 31, 32, 33 der Fußbedieneinheit 5 auf (Fig. 5).

In der vorliegenden Ausgestaltung ist vorgesehen, dass die elektrischen Kontakte 19, 20, 21 fest mit der Anlagefläche 14 verbunden sind. In alternativen Ausgestaltungen kann vorgesehen sein, dass die elektrischen Kontakte 19, 20, 21, die als Stiftkontakte ausgebildet sind, auch als federnde elektrische Kontakte ausgebildet sein können.

Die elektrischen Kontakte 19, 20, 21 sind elektrisch isoliert gegenüber der Seitenwand 13 angeordnet. Die elektrischen Kontakte 19, 20, 21 sind an die Ladeeinheit 17 angeschlossen.

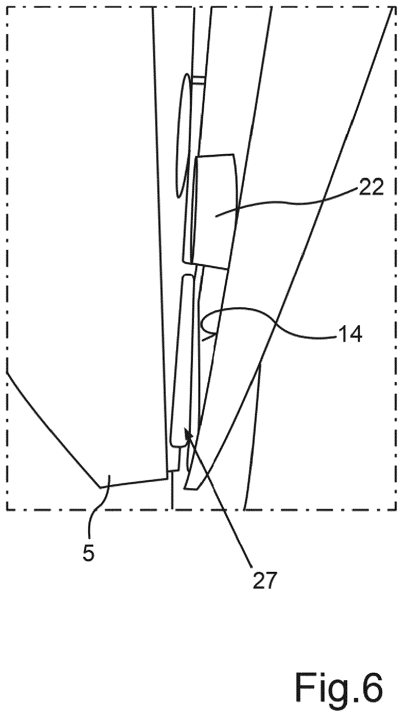

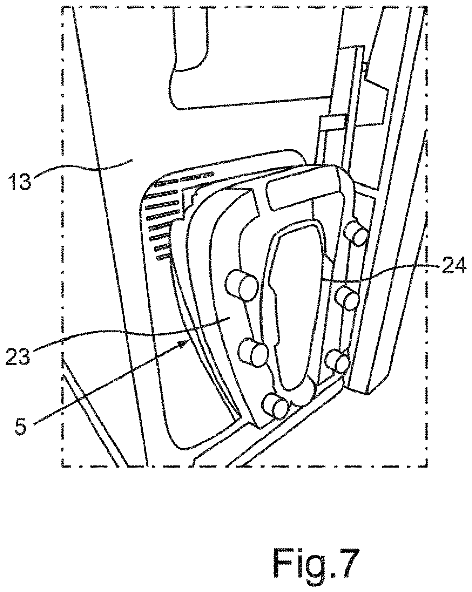

Fig. 5 zeigt in einer schematischen Draufsicht einen Ausschnitt einer Rückseite der Fußbedieneinheit 5. Die Fußbedieneinheit 5 weist ein Gehäuse 23 (Fig. 7), einen im Gehäuse 23 angeordneten elektrischen Energiespeicher 6 (Fig. 1), ein mittels eines Fußes betätigbares Stellglied 24 (Fig. 1), eine Kommunikationseinheit 26 zum drahtlosen Kommunizieren über Funk mit der Konsole 4 des ophthalmochirurgischen Systems 1 sowie eine Aufladeeinheit 25 zum Aufladen des elektrischen Energiespeichers 6 auf (Fig. 1). Das Gehäuse 23 weist einen Verbindungsbereich 27 zum lösbaren Anordnen der Fußbedieneinheit 5 in einer Nichtgebrauchsstellung an der Anlagefläche 14 der Konsole 4 auf (Fig. 6).

Der Verbindungsbereich 27 weist eine fußbedieneinheitsseitige Verbindungseinheit 36 zum lösbaren Verbinden der Fußbedieneinheit 5 mit der Konsole 4 auf. Der Verbindungsbereich 27 weist zwei Aufnahmeöffnungen 30 zum Aufnehmen von entsprechenden Haltestiften 16 der Konsole 4 auf, die voneinander zumindest horizontal beabstandet angeordnet sind. Dadurch kann die Fußbedieneinheit 5 auf einfache Weise an der Konsole 4 angeordnet werden, wobei die Haltestifte 16 in die als Sacköffnungen ausgebildeten Aufnahmeöffnungen 30 eingeführt werden. Durch die gleichzeitige geneigte Lage der Anlagefläche 14 kann somit durch die Gewichtskraft der Fußbedieneinheit 5 eine zuverlässige Verbindung mit der Konsole 4 erreicht werden, die lösbar ist.

Die Aufladeeinheit 25 weist einen Aufladeanschluss 28 auf, der die drei am Verbindungsbereich 27 angeordneten elektrischen Gegenkontakte 31, 32, 33 zum Kontaktieren von entsprechenden elektrischen Kontakten 19, 20, 21 der Konsole 4 aufweist. Die elektrischen Gegenkontakte 31, 32, 33 sind vorliegend als flächige, im Wesentlichen kreisförmige Kontaktplättchen ausgebildet, die über nicht dargestellte elektrische Leitungen an die Aufladeeinheit 25 angeschlossen sind.

Der Verbindungsbereich 27 weist das Plateau 35 zum Anordnen in einer durch den Kragen 22 bereitgestellten Ausnehmung auf. Die elektrischen Gegenkontakte 31, 32, 33 sind im Bereich einer Plateauebene 34 des Plateaus 35 angeordnet. Die Anordnung entspricht der Anordnung der elektrischen Kontakte 19, 20, 21, sodass eine elektrische Verbindung durch ein jeweiliges Kontaktieren erreicht werden kann, wenn die Fußbedieneinheit 5 an der Konsole 4 angeordnet ist. Sie sind vorliegend federnd gelagert, können in alternativen Ausgestaltungen jedoch auch fest mit dem Plateau 35 verbunden sein.

Durch die Erfindung kann insbesondere die Ausstattung eines modernen Arbeitsplatzes zur Kataraktoperation erreicht werden. Die Einheiten beziehungsweise Systeme des ophthalmochirurgischen Systems sind untereinander vernetzt, um eine gute Performance und eine einfache, intuitive Bedienung zu ermöglichen. Die Erfindung erlaubt es, Kabelverbindungen oder Schlauchverbindungen für Fluide zumindest teilweise zu vermeiden. Insbesondere elektrische Kabelverbindungen sowie auch Kommunikationsleitungen können nahezu vollständig vermieden werden. Zu diesem Zweck ist für das Fußbediengerät 5, auch Fußschaltpedal (FSP) genannt, eine drahtlose Kommunikationsverbindung zur Konsole 4 vorgesehen. Dies erfordert jedoch, dass das Fußbediengerät beziehungsweise die Fußbedieneinheit 5 auch eine zumindest teilweise unabhängige Energieversorgung aufweist, weshalb der elektrische Energiespeicher 6 vorgesehen ist, der üblicherweise als Akkumulator ausgebildet ist.

Außerhalb des bestimmungsgemäßen Betriebs des ophthalmochirurgischen Systems 1 ist das Fußbediengerät 5 durch die Erfindung der Konsole 4 zugeordnet lagerbar. Durch die vorgenannte Verbindungstechnik kann außerhalb des bestimmungsgemäßen Betriebs eine handhabbare Einheit aus der Konsole 4 und der Fußbedieneinheit 5 geschaffen werden, sodass für die Bereitstellung der entsprechenden Geräte für eine ophthalmochirurgische Operation auf einfache Weise bereitgestellt werden kann.

Darüber hinaus ermöglicht es die Erfindung, das Fußbediengerät 5, insbesondere dessen Energiespeicher 6, mit elektrischer Energie zu versorgen, wenn es in der Nichtgebrauchsstellung an der Konsole 4 angeordnet ist. Durch die hier vorgeschlagene elektrische Kontaktverbindung kann eine zuverlässige leitungsgebundene, zugleich aber kabellose elektrische Verbindung erreicht werden, sodass auf einfache Weise auch eine große Energiemenge in kurzer Zeit übertragen werden kann. Dabei können Störungen, wie sie beispielsweise bei einer Energieübertragung unter Nutzung von magnetischen Wechselfeldern auftreten können, weitgehend vermieden werden. Dies ist besonders für den medizinischen Bereich wichtig.

Die elektrischen Gegenkontakte 31, 32, 33 sind vorliegend in das Gehäuse 23 an einer Unterseite eingeklebt. Alternativ können sie auch eingespritzt werden, wenn das Gehäuse 23 zum Beispiel aus einem Kunststoff gebildet ist.

Vorliegend ist vorgesehen, dass die elektrische Spannung zwischen den Gegenelektroden 31, 32, 33 beziehungsweise den elektrischen Kontakten 19, 20, 21 etwa 15 V beträgt. Hierbei handelt es sich um eine Gleichspannung. Ein Ladestrom kann zum Beispiel etwa 1 A betragen. Darüber hinaus kann eine Schutzfunktion über den jeweils dritten elektrischen Kontakt 19 beziehungsweise elektrischen Gegenkontakt 31 erreicht werden, sodass die Versorgungsspannung von 15 V nur dann zur Verfügung gestellt wird, wenn ein elektrischer Kontakt über diese beiden elektrischen Kontakte 19, 31 hergestellt ist. Kann ein elektrischer Kontakt über diese Kontakte nicht hergestellt werden, kann vorgesehen sein, dass die elektrische Spannung auf etwa 3,3 V begrenzt wird oder sogar vollständig abgeschaltet wird. Dies kann durch die Ladeeinheit 17 erfasst werden, zu welchem Zweck die Aufladeeinheit 25 den elektrischen Gegenkontakt 31 mit einem entsprechenden Signal beaufschlagt. Die Ladefunktion kann dann zum Beispiel über die elektrischen Kontakte 20, 21 beziehungsweise 32, 33 erfolgen. Dadurch kann zum Beispiel auch erreicht werden, dass die elektrischen Kontakte 20, 21 nur dann mit elektrischer Spannung beaufschlagt werden, wenn die Ladeeinheit 17 einen entsprechenden Anschluss durch Anordnen der Fußbedieneinheit 5 an der Konsole 4 detektiert.

Darüber hinaus kann vorgesehen sein, dass die elektrische Spannung als Schutzkleinspannung bereitgestellt wird. Dadurch kann eine erhöhte Sicherheit erreicht werden.

Der Kragen 22 wirkt mit dem Plateau 35 zusammen, wenn die Fußbedieneinheit 5 an der Konsole 4 angeordnet ist. Unter anderem kann hierdurch eine Zentrierung erreicht werden, wenn eine Innenkontur der durch den Kragen 22 bereitgestellten Ausnehmung einer Außenkontur des Plateaus 35 angepasst gewählt ist. Dadurch kann auch eine Zentrierung der elektrischen Kontakte beziehungsweise elektrischen Gegenkontakte erreicht werden. Darüber hinaus kann der Kragen 22 die elektrischen Kontakte 19 bis 21 vor äußeren mechanischen Einwirkungen schützen.

Darüber hinaus wird vorgeschlagen, dass die Ladeeinheit 17 ausgebildet ist, ein elektrisches Potential an wenigstens einem der elektrischen Kontakte 19, 20, 21 auszuwerten, um ein Anordnen der Fußbedieneinheit 5 an der Konsole 4 zu detektieren. Dies kann dazu genutzt werden, dass die Ladeeinheit 17 die elektrischen Kontakte 19, 20, 21 nur dann mit einer elektrischen Spannung beaufschlagt, wenn eine entsprechende Anordnung der Fußbedieneinheit 5 an der Konsole 4 detektiert worden ist. Dadurch kann die elektrische Sicherheit verbessert werden. Zum Detektieren können natürlich auch Potentialdifferenzen zwischen Paaren von den elektrischen Kontakten 19, 20, 21 ausgewertet werden.

Die Ausführungsbeispiele dienen ausschließlich der Erläuterung der Erfindung und sollen diese nicht beschränken.

### Bezugszeichenliste

- 1: ophthalmochirurgisches System
- 2: Auge
- 3: Handstück
- 4: Konsole
- 5: Fußbedieneinheit
- 6: Energiespeicher
- 7: Steuereinheit
- 8: Versorgungseinheiten
- 9: Irrigationsleitung
- 10: Aspirationsleitung
- 11: Funkverbindung
- 12: Konsolengehäuse
- 13: Seitenwand
- 14: Anlagefläche
- 15: Verbindungseinheit
- 16: Haltestift
- 17: Ladeeinheit
- 18: Ladeanschluss
- 19: elektrischer Kontakt
- 20: elektrischer Kontakt
- 21: elektrischer Kontakt
- 22: Kragen
- 23: Gehäuse
- 24: Stellglied
- 25: Aufladeeinheit
- 26: Kommunikationseinheit
- 27: Verbindungsbereich
- 28: Aufladeanschluss
- 30: Aufnahmeöffnung
- 31: elektrischer Gegenkontakt
- 32: elektrischer Gegenkontakt
- 33: elektrischer Gegenkontakt
- 34: Plateauebene
- 35: Plateau
- 36: Verbindungseinheit

## Patentansprüche

1. Konsole (4) für ein medizinisches Behandlungssystem, insbesondere ein ophthalmochirurgisches System (1) zur Behandlung eines Auges (2), mit einem Konsolengehäuse (12) und einer Steuereinheit (7) zum Steuern eines medizinischen Behandlungsinstruments (3), wobei eine Fußbedieneinheit (5) drahtlos mit der Steuereinheit (7) koppelbar ist, und das Konsolengehäuse (12) wenigstens eine Seitenwand (13) aufweist,
**dadurch gekennzeichnet, dass**
die Seitenwand (13) eine Anlagefläche (14) zum Anordnen der Fußbedieneinheit (5) in einer Nichtgebrauchsstellung aufweist, wobei die Anlagefläche (14) zumindest im Bereich der Anordnung der Fußbedieneinheit (5) gegenüber einer horizontalen Ebene geneigt ist, um beim Anordnen der Fußbedieneinheit (5) an der Anlagefläche (14) durch die Gewichtskraft der Fußbedieneinheit (5) ein Anliegen der Fußbedieneinheit (5) an der Anlagefläche (14) zu bewirken, wobei die Seitenwand (13) im Bereich der Anlagefläche (14) eine konsolenseitige Verbindungseinheit (15) zum lösbaren Verbinden der Fußbedieneinheit (5) mit der Konsole (4) aufweist, wobei die Konsole (4) eine Ladeeinheit (17) zum Aufladen eines elektrischen Energiespeichers (6) der Fußbedieneinheit (5) aufweist, wobei die Anlagefläche (14) einen Ladeanschluss (18) mit wenigstens zwei elektrischen Kontakten (19, 20, 21) zum Kontaktieren von entsprechenden Gegenkontakten der Fußbedieneinheit (5) aufweist, wobei die Anlagefläche (14) einen aus der Anlagefläche (14) hervorragenden umlaufenden Kragen (22) aufweist, der die elektrischen Kontakte (19, 20, 21) umgibt, wobei die elektrischen Kontakte (19, 20, 21) vorzugsweise als federnde elektrische Stiftkontakte ausgebildet sind, wobei die konsolenseitige Verbindungseinheit (15) wenigstens zwei Haltestifte (16) zum Anordnen in entsprechenden Aufnahmeöffnungen der Fußbedieneinheit (5) aufweist, die voneinander zumindest horizontal beabstandet angeordnet sind.

2. Konsole nach Anspruch 1,
**dadurch gekennzeichnet, dass**
die Ladeeinheit (17) ausgebildet ist, ein elektrisches Potential an wenigstens einem der elektrischen Kontakte (19, 20, 21) auszuwerten, um ein Anordnen der Fußbedieneinheit (5) an der Konsole (4) zu detektieren.

3. Fußbedieneinheit (5) für ein medizinisches Behandlungssystem, insbesondere ein ophthalmochirurgisches System (1) zur Behandlung eines Auges (2), mit einem Gehäuse (23), einem im Gehäuse (23) angeordneten elektrischen Energiespeicher (6), einem mittels eines Fußes betätigbaren Stellglied (7), einer Kommunikationseinheit (26) zum drahtlosen Kommunizieren mit einer Konsole (4) des medizinischen Behandlungssystems (1) sowie einer Aufladeeinheit (25) zum Aufladen des elektrischen Energiespeichers (6),
**dadurch gekennzeichnet, dass**
das Gehäuse (23) einen Verbindungsbereich (27) zum lösbaren Anordnen der Fußbedieneinheit (5) in einer Nichtgebrauchsstellung an einer Anlagefläche (14) der Konsole (4) aufweist, wobei der Verbindungsbereich (27) eine fußbedieneinheitsseitige Verbindungseinheit (36) zum lösbaren Verbinden der Fußbedieneinheit (5) mit der Konsole (4) aufweist, wobei der Verbindungsbereich (27) wenigstens zwei Aufnahmeöffnungen (30) zum Aufnehmen von entsprechenden Haltestiften (16) der Konsole (4) aufweist, die voneinander zumindest horizontal beabstandet angeordnet sind, wobei die Aufladeeinheit (25) einen Aufladeanschluss (28) mit wenigstens zwei am Verbindungsbereich (27) angeordneten elektrischen Gegenkontakten (31, 32, 33) zum Kontaktieren von entsprechenden elektrischen Kontakten (19, 20, 21) der Konsole (4) aufweist.

4. Fußbedieneinheit nach Anspruch 3,
**dadurch gekennzeichnet, dass**
der Verbindungsbereich (27) ein Plateau (35) zum Anordnen in einer durch einen Kragen (22) bereitgestellten Ausnehmung aufweist, wobei die elektrischen Gegenkontakte (31, 32, 33) im Bereich einer Plateauebene (34) des Plateaus (35) angeordnet sind.

5. Medizinisches Behandlungssystem, insbesondere ophthalmochirurgisches System (1) zur Behandlung eines Auges (2), mit zumindest:
- einem medizinischen Behandlungsinstrument (3),
- einer Konsole (4) zum Anschließen und Betreiben des medizinischen Behandlungsinstruments (3) in einem bestimmungsgemäßen Betrieb,
- einer Fußbedieneinheit (5) zum zumindest teilweisen Steuern des bestimmungsgemäßen Betriebs des medizinischen Behandlungsinstruments (3), wobei die Fußbedieneinheit (5) drahtlos mit der Konsole (4) koppelbar ist und einen elektrischen Energiespeicher (6) zum Versorgen der Fußbedieneinheit (5) mit elektrischer Energie zumindest während des bestimmungsgemäßen Betriebs des medizinischen Behandlungssystems (1) aufweist,
**dadurch gekennzeichnet, dass**
wenigstens die Konsole (4) nach einem der vorhergehenden Ansprüche 1 oder 2 oder die Fußbedieneinheit (5) nach einem der vorhergehenden Ansprüche 3 oder 4 ausgebildet ist.

## Claims

1. Console (4) for a medical treatment system, in particular an ophthalmosurgical system (1) for the treatment of an eye (2), with a console housing (12) and a control unit (7) for controlling a medical treatment instrument (3), wherein a foot-operated control unit (5) can be wirelessly coupled to the control unit (7), and the console housing (12) has at least one side wall (13),
**characterized in that**
the side wall (13) has a bearing surface (14) for arranging the foot-operated control unit (5) in a not-in-use position, wherein the bearing surface (14) is inclined with respect to a horizontal plane, at least in the region where the foot-operated control unit (5) is arranged, in order when arranging the foot-operated control unit (5) on the bearing surface (14) to bring about bearing of the foot-operated control unit (5) against the bearing surface (14) by the weight of the foot-operated control unit (5), wherein the side wall (13) has in the region of the bearing surface (14) a console-side connecting unit (15) for releasably connecting the foot-operated control unit (5) to the console (4), wherein the console (4) has a charging unit (17) for charging an electrical energy store (6) of the foot-operated control unit (5), wherein the bearing surface (14) has a charging connection (18) with at least two electrical contacts (19, 20, 21) for contacting corresponding mating contacts of the foot-operated control unit (5), wherein the bearing surface (14) has a peripheral collar (22), which projects from the bearing surface (14) and surrounds the electrical contacts (19, 20, 21), wherein the electrical contacts (19, 20, 21) are preferably formed as resilient electrical pin contacts, wherein the console-side connecting unit (15) has at least two retaining pins (16) for arranging in corresponding receiving openings of the foot-operated control unit (5), which are arranged at least horizontally spaced-apart from one another.

2. Console according to Claim 1,
**characterized in that**
the charging unit (17) is designed to evaluate an electrical potential at at least one of the electrical contacts (19, 20, 21) in order to detect that the foot-operated control unit (5) is arranged on the console (4).

3. Foot-operated control unit (5) for a medical treatment system, in particular an ophthalmosurgical system (1) for the treatment of an eye (2), with a housing (23), an electrical energy store (6) arranged in the housing (23), an actuating element (7) which can be actuated by means of a foot, a communication unit (26) for wireless communication with a console (4) of the medical treatment system (1) and also a charging unit (25) for charging the electrical energy store (6), **characterized in that**
the housing (23) has a connecting region (27) for releasably arranging the foot-operated control unit (5) in a not-in-use position on a bearing surface (14) of the console (4), wherein the connecting region (27) has a connecting unit (36) on the foot-operated control unit side for releasably connecting the foot-operated control unit (5) to the console (4), wherein the connecting region (27) has at least two receiving openings (30) for receiving corresponding retaining pins (16) of the console (4), which are arranged at least horizontally spaced-apart from one another, wherein the charging unit (25) has a charging connection (28) with at least two electrical mating contacts (31, 32, 33), arranged at the connecting region (27), for contacting corresponding electrical contacts (19, 20, 21) of the console (4).

4. Foot-operated control unit according to Claim 3, **characterized in that**
the connecting region (27) has a plateau (35) for arranging in a clearance provided through a collar (22), wherein the electrical mating contacts (31, 32, 33) are arranged in the region of a plateau plane (34) of the plateau (35).

5. Medical treatment system, in particular ophthalmosurgical system (1) for the treatment of an eye (2), with at least:
- a medical treatment instrument (3),
- a console (4) for connecting and operating the medical treatment instrument (3) during an as-intended operating mode,
- a foot-operated control unit (5) for at least partially controlling the as-intended operating mode of the medical treatment instrument (3), wherein the foot-operated control unit (5) can be wirelessly coupled to the console (4) and has an electrical energy store (6) for supplying the foot-operated control unit (5) with electrical energy, at least during the as-intended operating mode of the medical treatment system (1),
**characterized in that**
at least the console (4) is designed according to either of the preceding Claims 1 and 2 or the foot-operated control unit (5) is designed according to either of the preceding Claims 3 and 4.

## Revendications

1. Console (4) pour un système de traitement médical, en particulier un système de chirurgie ophtalmologique (1) destiné au traitement d'un œil (2), comprenant un boîtier de console (12) et une unité de commande (7) pour commander un instrument de traitement médical (3), dans laquelle une unité de commande au pied (5) peut être couplée sans fil à l'unité de commande (7), et le boîtier de console (12) présente au moins une paroi latérale (13),
**caractérisée en ce que** la paroi latérale (13) présente une surface de contact (14) pour disposer l'unité de commande au pied (5) dans une position de non-utilisation, dans laquelle, au moins dans la zone où est disposée l'unité de commande au pied (5), la surface de contact (14) est inclinée par rapport à un plan horizontal afin de provoquer par le poids de l'unité de commande au pied (5) un contact de l'unité de commande au pied (5) avec la surface de contact (14) lorsque l'unité de commande au pied (5) est disposée sur la surface de contact (14), dans laquelle la paroi latérale (13) présente dans la zone de la surface de contact (14) une unité de raccordement (15) côté console pour le raccordement amovible de l'unité de commande au pied (5) à la console (4), dans laquelle la console (4) présente une unité de charge (17) pour recharger un accumulateur d'énergie électrique (6) de l'unité de commande au pied (5), dans laquelle la surface de contact (14) présente un port de charge (18) pourvu d'au moins deux contacts électriques (19, 20, 21) pour la mise en contact avec des contre-contacts correspondants de l'unité unité de commande au pied (5), dans laquelle la surface de contact (14) présente une collerette périphérique (22) faisant saillie à partir de la surface de contact (14) et qui entoure les contacts électriques (19, 20, 21), dans laquelle les contacts électriques (19, 20, 21) sont réalisés de préférence sous la forme de broches contact électriques élastiques, dans laquelle l'unité de raccordement (15) côté console présente au moins deux goupilles de fixation (16) destinées à être disposées dans des ouvertures de réception correspondantes de l'unité de commande au pied (5) et qui sont disposées de manière au moins horizontalement espacée l'une de l'autre.

2. Console selon la revendication 1, **caractérisée en ce que** l'unité de charge (17) est réalisée pour évaluer un potentiel électrique au niveau d'au moins l'un des contacts électriques (19, 20, 21) afin de détecter le fait que l'unité de commande au pied (5) est disposée sur la console (4).

3. Unité de commande au pied (5) pour un système de traitement médical, en particulier un système de chirurgie ophtalmologique (1) destiné au traitement d'un œil (2), comprenant un boîtier (23), un accumulateur d'énergie électrique (6) disposé dans le boîtier (23), un actionneur (7) pouvant être actionné au pied, une unité de communication (26) pour la communication sans fil avec une console (4) du système de traitement médical (1), ainsi qu'une unité de charge (25) pour recharger l'accumulateur d'énergie électrique (6),
**caractérisée en ce que** le boîtier (23) présente une zone de raccordement (27) pour la disposition amovible de l'unité de commande au pied (5) dans une position de non-utilisation sur une surface de contact (14) de la console (4), dans laquelle la zone de raccordement (27) présente une unité de raccordement (36) côté unité de commande au pied pour le raccordement amovible de l'unité de commande au pied (5) à la console (4), dans laquelle la zone de raccordement (27) présente au moins deux ouvertures de réception (30) pour recevoir des goupilles de fixation (16) correspondantes de la console (4) qui sont disposées de manière au moins horizontalement espacée l'une de l'autre, dans laquelle l'unité de charge (25) présente un port de charge (28) pourvu d'au moins deux contre-contacts électriques (31, 32, 33) disposés dans la zone de raccordement (27) pour la mise en contact avec des contacts électriques correspondants (19, 20, 21) de la console (4).

4. Unité de commande au pied selon la revendication 3, **caractérisée en ce que** la zone de raccordement (27) présente un plateau (35) destiné à être disposé dans un évidement fourni par une collerette (22), dans laquelle les contre-contacts électriques (31, 32, 33) sont disposés dans la zone d'un plan de plateau (34) du plateau (35).

5. Système de traitement médical, en particulier système de chirurgie ophtalmologique (1) pour le traitement d'un œil (2), comprenant au moins :
- un instrument de traitement médical (3),
- une console (4) pour raccorder et faire fonctionner l'instrument de traitement médical (3) en fonctionnement conforme,
- une unité de commande au pied (5) pour la commande au moins partielle du fonctionnement conforme de l'instrument de traitement médical (3), l'unité de commande au pied (5) pouvant être couplée sans fil à la console (4) et présentant un accumulateur d'énergie électrique (6) pour alimenter l'unité de commande au pied (5) en énergie électrique au moins pendant le fonctionnement conforme du système de traitement médical (1),
**caractérisé en ce qu'**au moins la console (4) est réalisée selon l'une quelconque des revendications précédentes 1 ou 2 ou l'unité de commande au pied (5) est réalisée selon l'une quelconque des revendications précédentes 3 ou 4.
